(19) Europäisches Patentamt
European Patent Office
Office européen des brevets

(11) Publication number: 0 113 185 B2

(12) NEW EUROPEAN PATENT SPECIFICATION

(45) Date of publication of the new patent specification: 24.07.91 Bulletin 91/30

(51) Int. Cl.[5]: C07C 17/14, C07C 25/13

(21) Application number: 83307238.2

(22) Date of filing: 28.11.83

# (54) Selective bromination process.

(30) Priority: 06.01.83 GB 8300281

(43) Date of publication of application: 11.07.84 Bulletin 84/28

(45) Publication of the grant of the patent: 09.04.86 Bulletin 86/15

(45) Mention of the opposition decision: 24.07.91 Bulletin 91/30

(84) Designated Contracting States: CH DE FR GB IT LI NL

(56) References cited:
EP-A- 0 009 787
EP-A- 0 011 281
DE-A- 2 844 270
DE-C- 478 084
DE-C- 2 716 896
DE-C-47 808 4

(56) References cited:
GB-A- 2 036 016
US-A- 3 714 278
US-A- 4 240 983
ANNALI DI CHIMICA 41, (1951), p. 491-498
HOUBEN-WEYL, Methoden der organischen Chemie, 4. Auflage, Band V/3, p. 519-20
Ullman's Enzyklopädie der technischen Chemie, 4. Auflage, Band 9, p. 529-30

(73) Proprietor: IMPERIAL CHEMICAL INDUSTRIES PLC
Imperial Chemical House, Millbank
London SW1P 3JF (GB)

(72) Inventor: Hann, Richard Anthony
5 Marling Close
Frodsham Warrington WA6 6AY (GB)

(74) Representative: Houghton, Malcolm John et al
Imperial Chemical Industries PLC Legal Department: Patents PO Box 6
Welwyn Garden City Herts, AL7 1HD (GB)

EP 0 113 185 B2

## Description

This invention relates to a process for the selective bromination of alkyl aromatic hydrocarbons and their halogenated substitution products. The brominated products are useful chemical intermediates in the preparation of a variety of compounds, including compounds having insecticidal properties.

It is well known that the alkyl side chain of an alkyl aromatic hydrocarbon can be brominated by contacting the hydrocarbon with bromine in the presence of a catalyst or under the influence of natural or artificial light. Under conventional conditions of bromination it is difficult to secure selective production of the desired partially brominated product as this can undergo further bromination when it is formed. Hence, when equimolar quantities of bromine and the hydrocarbon are reacted, the reaction product may be a mixture of the mono-, di- and un-brominated hydrocarbons in approximately the statistically expected amounts of 50%, 25%, and 25%, respectively.

European Patent Application No. 0009787 relates to a process for the mono-halogenation of alkylbenzenes in the alpha-position. In this process there is an excess of alkylbenzene and continuous reaction with a halogen, the halogen being in the gaseous form. The halogenated alkylbenzene is continuously withdrawn from the reaction area.

US 4,240,983 discloses a process for the preparation of monohalogenated ketones by vaporising the ketone and reacting halogen with condensed ketone on the collector. The halogen is introduced into the reaction in the gaseous state.

UK Patent Application No. 2036016 discloses a process for the halogenation of aliphatic ethers and ketones. The halogen is introduced directly into the still-head reactor, most conveniently in the gaseous form, so that the reaction takes place at still head temperatures and in the presence of bromine and reactant vapour.

A process aimed at reducing the over-halogenation of alkyl aromatic hydrocarbons and their halogenated substitution products is described in German Patent Specification No. 478084. In this process a halogen is allowed to act on the vapour of the material to be halogenated under the influence of daylight or artificial light in a halogenation chamber, the temperature of which is kept below the boiling point of the desired product. The product condenses when formed and is immediately removed from the halogenating chamber to protect it from further halogenation. The preparation of benzyl bromide is exemplified.

It has been found that when brominations are carried out by this process, using as the halogenation chamber the still-head of a distillation system such as that described in UK Patent Specification No. 2036016A, a considerable amount of over-bromination still occurs. The present invention is aimed at reducing over-bromination still further.

According to the present invention there is provided an improved process for the selective mono-bromination of an alkyl aromatic hydrocarbon or a halogenated substitution product thereof (hereinafter called "the hydrocarbon") by contacting the hydrocarbon with bromine in the still head of a distillation system wherein the hydrocarbon is boiled under reflux and the partially brominated product so formed continuously removed from the still head as a relatively high boiling component, characterised in that condensed hydrocarbon and bromine liquid are mixed, in a mixing zone prior to passing through a reaction zone on the way to the still head, in the liquid phase.

Conveniently, liquid bromine is introduced to the still head through a side-arm below a condenser or possibly through the condenser itself, and allowed to mix with the condensing hydrocarbon. The mixed reactants are passed through a reaction zone in liquid form before the partially brominated product so formed is allowed to drain from the still head. Preferably the reaction zone is illuminated with ultraviolet or visible light of wavelength shorter than 550 nm and the reactants well mixed before entering the illuminated reaction zone.

The best results have been achieved when the rate of addition of the bromine has been controlled so that the bromine is contacted with the hydrocarbon in a molar ratio below 1 to 6 of bromine to hydrocarbon.

The process of the invention has been found advantageously applicable to the mono-bromination of ring halogenated alkyl aromatic hydrocarbons and particularly to toluene and p-xylene products of the formula :

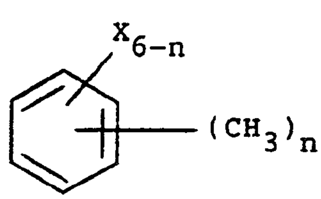

in which X is F or Cl, n is 1 or 2 and when n is 2 the methyl groups are in positions ortho or para to each other.

As examples of compounds which may be obtained, there are mentioned, in particular, pentafluorobenzyl

bromide and tetrafluoro-p-xylyl bromide.

The invention will now be illustrated, exemplified and compared with a vapour phase process with reference to the accompanying drawings in which

Figure 1 is a diagrammatic illustration of apparatus suitable for carrying out a liquid phase bromination process ; and

Figure 2 is a diagrammatic illustration of apparatus suitable for carrying out a gas phase bromination process.

In Figure 1, a distillation system comprises a heated flask 11 surmounted by a distillation column 17, a condenser 13 and, in a return loop 19, a mixing zone 14 and a reaction chamber 15 through which condensate will pass on its return to flask 11. A side-arm 16 provides an inlet for bromine to the mixing zone 14.

In use, the hydrocarbon to be brominated is heated to reflux in flask 11 and liquid bromine added slowly through the side-arm 16. The condensed hydrocarbon and bromine liquid, after being well mixed in the mixing zone 14, flow through the reaction chamber 15, which is irradiated using a medium pressure mercury vapour lamp (not shown). The higher boiling brominated hydrocarbon which is formed returns to the flask 11 via column 17 and unreacted materials are recycled. Uncondensed hydrogen bromide produced during bromination is removed via the condenser 13.

In a modified distillation system shown in Figure 2, still head 51 is surmounted by a condenser 53 and fed from dropping funnel 55 via line 57 of which part 59 is wrapped with heating tape (not shown) and part 61 serviced by a hot air blower (not shown).

In use, liquid bromine introduced into line 57 through dropping funnel 55 is vaporised by the application of heat to the line and mixes in the vapour phase with refluxing hydrocarbon in still head 51 which is irradiated by the mercury vapour lamp.

## Example 1

### Liquid phase bromination of tetrafluoro-p-xylene

Tetrafluoro-p-xylene (36.1 g 98% pure p-isomer) was heated to reflux in flask 11 and bromine (10.5 ml) added slowly through the side-arm 16 over 3 hours, after which the still-head temperature was 158°C. A brown residue (49g) in flask 11 was extracted with aqueous sodium bicarbonate and sodium thiosulphate and the organic layer (47 g) separated, dried over magnesium sulphate and filtered.

Including material recovered by washing the magnesium sulphate with methylene chloride, the final yield of organic material was 46.2 g. Analysis by Gas Chromatography showed this material to comprise 13.7% tetrafluoro-p-xylene, 80.0% tetrafluoro-p-xylyl bromide and 3.95% tetrafluoro-p-xylylene dibromide ; a yield of 71% tetrafluoro-p-xylyl bromide.

Crystalline material deposited from the organic mixture was shown to be tetrafluoro-p-xylyl bromide, having a melting point of 50°C and a boiling point of 207°C.

### Elemental Analysis

| | |
|---|---|
| C | 35.9% |
| H | 1.9% |
| F | 30.0% |
| Br | 30.3% |

$C_8H_5F_4Br$ requires C 37.4%, H 2.0%, F 29.6%, Br 31.1%.

NMR

$^1H$; 90M Hz; $CDCl_3$/TMS; singlet 5.5 (2H)
singlet 7.7 (3H)

$^{19}F$ ; 84.6M Hz ; $CDCl_3/CFCl_3$ : doublet of quartets 145ppm.

Analysis of Mass Spectrometry supported identification.

## Example 2

Liquid phase bromination of pentafluorotoluene

Pentafluorotoluene (53.8 g) was heated to reflux in flask 11 and bromine (15 ml) added through the side-arm 16. The reflux temperature rose from 119°C to 130°C during the bromine addition and a further 1.5 ml bromine added until the temperature had reached 140°C. Refluxing was continued inadvertently for 1½ hours. A dark residue in flask 11 was combined with a small quantity of material in the recycle loop 15 and washed with aqueous sodium bicarbonate and sodium thiosulphate solutions. The organic layer was separated, dried over magnesium sulphate and filtered. The filter residue was washed with methylene chloride and the washings heated to evaporate the solvent. All recovered material was combined to give 72.1 g of a pale brown oil. Analysis by Gas Chromatography showed the oil to comprise 8.4% starting material, 91.6% pentafluorobenzyl bromide and a trace of a dibrominated compound.

Example 3

Liquid phase bromination of tetrafluoro-p-xylene

Example 1 was repeated except that

(a) the bromine addition was better controlled by using a dropping funnel with a PTFE screw control ;

(b) the bromine was added over 4 hours until the still-head temperature had risen form 150°C to 168°C ;

(c) quantities of reactants used were - tetrafluoro-p-xylene 62 g ; and bromine 19.2 ml ; and

(d) a compressed air jet was used to cool the parts of the still subject to heat from the UV lamp and in particular the recycle loop 15.

Product taken from the flask 11 was washed with aqueous sodium bicarbonate and sodium thiosulphate and dried over anhydrous magnesium sulphate yielding 83.4g. Further product (2.0 g) was obtained by washing the distillation apparatus with methylene chloride which was then dried and evaporated. Analysis of the combined product (85.4 g) gave its composition as 9% tetrafluoro-p-xylene, 90% tetrafluoro-p-xylyl bromide and 2.2% tetrafluoro-p-xylylene dibromide ; a yield of 85.6% tetrafluoro-p-xylyl bromide.

Finally, the tetrafluoro-p-xylyl bromide (50.7 g) was purified and separated as white crystals by recrystalisation.

Example 4 (Comparative only)

Gas phase bromination of tetrafluoro-p-xylene

Using the modified apparatus of Figure 2, tetrafluoro-p-xylene (25.2 g) was heated to reflux in flask 11 and bromine added slowly from a dropping funnel 55. Much of the bromine could be seen, through smoked glass, to be vaporised. After the addition of approximately 5.8 ml bromine over 70 minutes, no more material was seen refluxing at the top of the column and the reaction was stopped.

The product in flask 51 was extracted with aqueous sodium bicarbonate and sodium thiosulphate, when it became semi-solid. The apparatus was rinsed with methylene chloride and the rinsings evaporated on a steam bath to give a second crop of material which was added to the first. Analysis of the combined product (33.6 g) showed it to comprise 21.5% tetrafluoro-p-xylene, 65.1% tetrafluoro-p-xylyl bromide and 12.2% tetrafluoro-p-xylene dibromide ; a yield of 60.3% tetrafluoro-p-xylyl bromide.

Discussion of results

A comparison of Examples 1 and 3 with Example 4 shows considerably higher levels of dibromide formation (3.9% and 2.2% vs. 12.2%) using gas phase bromination which, it is concluded, is a less selective bromination technique.

**Claims**

1. A process for the selective mono-bromination of an alkyl aromatic hydrocarbon or a halogenated substitution product thereof (hereinafter called "the hydrocarbon") by contacting the hydrocarbon with bromine in the still head of a distillation system wherein the hydrocarbon is boiled under reflux and the partially brominated product so formed continuously removed from the still head as a relatively high-boiling component, characterised in that condensed hydrocarbon and bromine liquid are mixed, in a mixing zone prior to passing through a

reaction zone on the way to the still head, in the liquid phase.

2. A process according to claim 1 in which the bromine is contacted with the hydrocarbon in a molar ratio below 1 to 6 of bromine to hydrocarbon.

3. A process according to claim 1 or 2 in which the hydrocarbon has the formula :

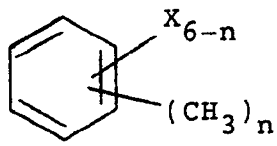

in which X is F or Cl, n is 1 or 2 and when n is 2 the methyl groups are in positions ortho or para to each other.

4. A process according to any one of the preceding claims in which pentafluorobenzyl bromide or tetrafluoro-p-xylyl bromide is obtained from pentafluorotoluene or tetrafluoro-p-xylene, respectively.

## Patentansprüche

1. Verfahren zur selektiven Monobromierung eines alkylsubstituierten aromatischen Kohlenwasserstoffs oder eines halogenierten Substitutionsprodukts desselben (in der Folge als der "Kohlenwasserstoff" bezeichnet) durch Inberührungbringen des Kohlenwasserstoffs im Kolonnenkopf eines Destillationssystems, in welchem der Kohlenwasserstoff unter Rückfluß am Sieden gehalten wird und das so gebildete teilweise bromierte Produkt aus dem Kolonnenkopf als verhältnismäßig hochsiedende Komponente kontinuierlich abgeführt wird, **dadurch gekennzeichnet**, daß kondensierter Kohlenwasserstoff und flüssiges Brom in der flüssigen Phase in einer Mischzone gemischt werden, bevor sie auf dem Weg zum Kolonnenkopf in eine Reaktionszone fließen.

2. Verfahren nach Anspruch 1, bei welchem das Brom mit dem Kohlenwasserstoff in einem Verhältnis von weniger als 1 bis 6 Mol Brom je Mol Kohlenwasserstoff in Berührung gebracht wird.

3. Verfahren nach Anspruch 1 oder 2, bei welchem der Kohlenwasserstoff die Formel

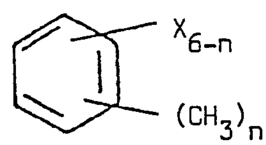

aufweist,worin X für F oder Cl steht, n für 1 oder 2 steht und, wenn n für 2 steht, die Methylgruppen sich in der Ortho- oder Parastellung zueinander befinden.

4. Verfahren nach einem der vorhergehenden Ansprüche, bei welchem Pentafluorobenzylbromid oder Tetrafluoro-p-xylylbromid aus Pentafluorotoluol bzw. Tetrafluoro-p-xylol erhalten wird.

## Revendications

1. Procédé de bromation sélective d'un hydrocarbure alkyl-aromatique ou d'un produit de substitution halogéné de cet hydrocarbure (que l'on appellera "hydrocarbure" ci-après) par mise en contact de l'hydrocarbure avec du brome dans la tête d'alambic d'un dispositif de distillation où l'hydrocarbure est maintenu à l'ébullition au reflux et le produit partiellement bromé ainsi formé est continuellement déchargé de la tête d'alambic comme composant de point d'ébullition relativement haut, caractérisé en ce que l'hydrocarbure condensé et du brome liquide sont mélangés, dans une zone de mélange avant de passer dans une zone de réaction sur le chemin de la tête d'alambic, en phase liquide.

2. Procédé suivant la revendication 1, dans lequel le brome est mis en contact avec l'hydrocarbure dans un rapport molaire brome :hydrocarbure inférieur à 1 :6.

3. Procédé suivant la revendication 1 ou 2, dans lequel l'hydrocarbure répond à la formule

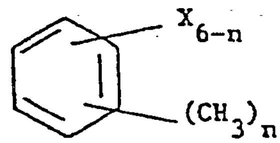

dans laquelle X représente F ou Cl, n a la valeur 1 ou 2 et lorsque n est égal à 2, les groupes méthyle sont en position ortho ou para l'un par rapport à l'autre.

4. Procédé suivant l'une quelconque des revendications précédentes, dans lequel du bromure de pentafluorobenzyle ou du bromure de tétrafluoro-p-xylyle est obtenu respectivement à partir de pentafluorotoluène ou de tétrafluoro-p-xylène.

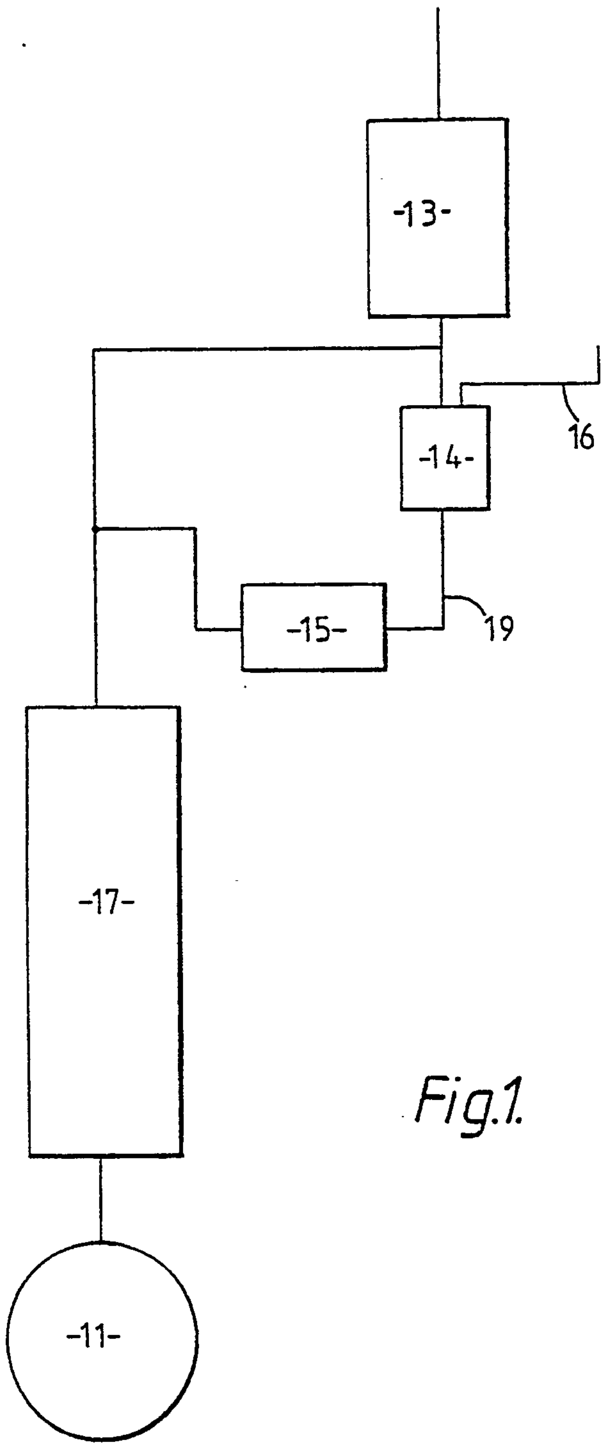


Fig.1.

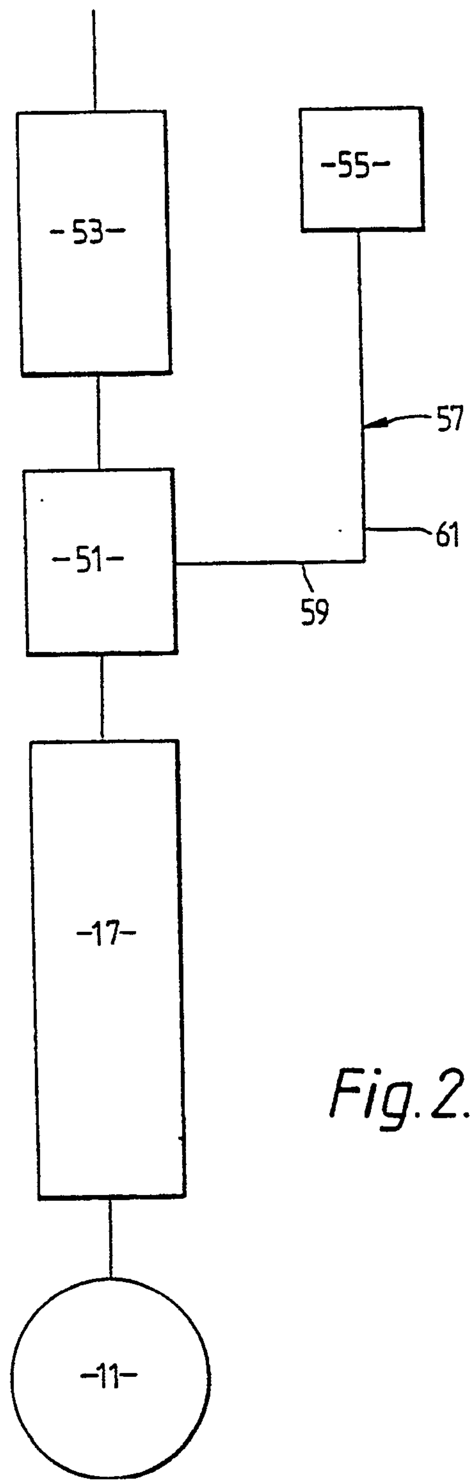


Fig.2.